# EUROPEAN PATENT APPLICATION

(11) **EP 4 681 675 A1**
(43) Date of publication of application: **21.01.2026**
(21) Application number: 24927709.6
(22) Date of filing: 10.12.2024
(51) Int. Cl.: A61B 34/00, A61B 34/30, A61B 17/56, A61B 90/50

(54) **MULTIPLE-DEGREE-OF-FREEDOM SURGICAL DEVICE AND OPERATING METHOD THEREOF**

(30) Priority: 27.05.2024 KR 20240068882
(71) Applicant: Curexo, Inc., Seoul 05814 (KR)
(72) Inventor: KIM, Young Hwan, Songpa-gu Seoul 05814 (KR); KIM, Yong Seok, Songpa-gu Seoul 05814 (KR); KIM, Si Jin, Songpa-gu Seoul 05814 (KR)
(74) Representative: Lavoix
(86) International application number: PCT/KR2024/096647
(87) International publication number: WO 2025/249690

(57) **Abstract**

A surgical device and a method of controlling the same are described. The surgical device includes: a mounting plate on which an end-effector including a tool is mounted, the mounting plate being placed on a plane parallel to both a first axis along which the tool extends and a second axis crossing the first axis; a first actuator on which the mounting plate is mounted, the first actuator causing the mounting plate to perform a first motion in a direction of the second axis; a second actuator configured to rotate the first actuator along a circumference of a third axis orthogonal to the first axis and the second axis to generate rotational power causing a second motion of the mounting plate; and a third actuator coupled to the second actuator and configured to transfer the rotational power from the second actuator to the first actuator and to cause a third motion in which the mounting plate is rotated along a circumference of a rotational shaft in a direction of the second axis.

## Description

### Technical Field

The present disclosure relates to a multi-degree of freedom surgical device used for joint surgery or the like, and a method of controlling the multi-degree of freedom surgical device, and more particularly, to a surgical device for aligning or positioning a surgical end-effector by 3 degrees of freedom (3DoF) or more and a method of controlling the surgical device.

### Background Art

Joint damage caused by various reasons is accompanied by deformation and functional loss as well as pain. Artificial joint surgery may be selected when a non-surgical method or even an initial surgical treatment by osteotomy or the like for a damaged joint is not effective.

Artificial joint surgery, which is one type of surgical operation, is performed by inserting an artificial replacement into a joint part, and for this, a portable saw driver or drill driver is used as a surgical device having a drill-type or saw-type end-effector, etc.

The saw driver is used to partially cut a bone of a joint part, and the drill driver is used to form a tunnel (hole) or fix a pin in a bone. For example, before a bone is cut by the saw driver, a tunnel is formed by the drill driver for insertion of a surgical pin, and the surgical pin is fixed therein, and in certain cases, the drill used to form the tunnel may remain as the pin.

During surgery, a stiff and damaged part is removed using a saw driver or the like from damaged joints, such as femur, tibia, or patella, and an artificial substitution, such as a finely manufactured artificial joint, is inserted and fixed into this surgical site.

To normally insert the artificial replacement into the surgical site, the position and direction of an end-effector need to be accurately maintained with respect to the surgical tube, so as to cut bone according to a pre-set surgical plan.

Though there is support of a so-called surgical navigation system, an end-effector of a previous low degree of freedom, for example, 2 degrees of freedom, cannot cope sufficiently well with a limited surgical space and restricted postures of a surgeon.

In this respect, it may be highly useful to develop a high degree of freedom surgical device, according to which a surgeon may have relatively increased freedom of posture, and a position and direction of an end-effector may match a corresponding surgical site according to a pre-set surgical plan.

### Disclosure of Invention

### Technical Problem

The present disclosure provides a surgical device having a high degree of freedom.

The present disclosure provides a surgical device for aligning and positioning an end-effector in a desired surgical position, regardless of a posture of a surgeon, and a method of driving the surgical device.

### Solution to Problem

A surgical device according to one or more embodiments includes:
a mounting plate on which an end-effector including a surgical tool extending in a first direction is mounted, the mounting plate being placed on a plane parallel to both a first axis in the first direction and a second axis in a second direction, the second direction crossing the first direction;
a first actuator including a first plate, on which the mounting plate is mounted to be capable of a reciprocating movement in the second direction, the first actuator causing the mounting plate to perform a first motion in the second direction with respect to the first plate;
a second actuator configured to rotate the first plate with respect to the first axis in the first direction to generate rotation to cause a second motion of the end-effector mounted on the mounting plate; and
a third actuator configured to make the first plate vertically rise up and fall down in a direction of a third axis orthogonal to both the first axis and the second axis to cause a third motion of the end-effector according to the vertical rising up and falling down, and to rotate the first plate with respect to the second axis in the second direction to cause a fourth motion of the end-effector according to the rotation.

In the surgical device according to one or more embodiments,
the first actuator may include: a first motor configured to drive the mounting plate on the first plate; and a first power transmission unit configured to transmit power of the first motor to operate the mounting plate through the first motor.

In the surgical device according to one or more embodiments,
the first power transmission unit may include: a first linear mover configured to convert rotary movement of the first motor into linear reciprocating movement; and a translational lever configured to transfer the reciprocating linear movement of the first linear mover to the mounting plate on the first plate to cause translational movement of the mounting plate.

In the surgical device according to one or more embodiments,
the linear movement of the first linear mover may be performed in a direction crossing the plane of the mounting plate, and the translational lever may be hinge-coupled to the mounting plate to convert the linear movement of the first linear mover into a direction of the translational movement of the mounting plate.

In the surgical device according to one or more embodiments,
the second actuator may include:
a second plate rotatably hinge-coupled to the first plate;
a second motor mount integrally coupled to the second plate;
a second motor mounted on the second motor mount;
a second linear mover configured to convert rotational force of the second motor into linear reciprocating movement; and
a rolling lever of which both ends are connected to the second linear mover and the first plate, the rolling lever being configured to cause the second motion of the first plate with respect to the second plate.

In the surgical device according to one or more embodiments,
the first, the second, and the third actuators may be arranged in a housing protecting the first to third actuators, and
the third actuator may include:
   a third plate, a position of which is fixed to the housing;
   a third motor and a fourth motor, configured to cause the third motion and the fourth motion of the first plate;
   a third motor mount configured to support the third motor and the fourth motor and integrally fixed to the third plate;
   a third linear mover and a fourth linear mover connected to the third motor and the fourth motor, respectively, and configured to convert rotary movement into linear reciprocating movement; and
   a third operating rod and a fourth operating rod connected to the third linear mover and the fourth linear mover, respectively, and configured to collaboratively cause the third motion and/or the fourth motion of the first plate.

In the surgical device according to one or more embodiments,
the third operating rod may be directly hinge-coupled to the first plate, and the fourth operating rod may be, through a link lever, hinge-coupled to the first plate.

In the surgical device according to one or more embodiments,
the first, the second, and the third actuators may be arranged in a housing protecting the first to third actuators, and
the third actuator may include:
   a third plate, a position of which is fixed with respect to the housing;
   a third motor and a fourth motor, configured to cause the third motion and the fourth motion of the first plate;
   a third motor mount configured to support the third motor and the fourth motor and integrally fixed to the third plate;
   a third linear mover and a fourth linear mover connected to the third motor and the fourth motor, respectively, and configured to convert rotary movement into linear reciprocating movement; and
   a third operating rod and a fourth operating rod connected to the third linear mover and the fourth linear mover, respectively, and configured to collaboratively cause the third motion and/or the fourth motion of the first plate.

In the surgical device according to one or more embodiments,
the third operating rod may be directly hinge-coupled to the first plate, and the fourth operating rod may be, through a link lever, hinge-coupled to the first plate.

In the surgical device according to one or more embodiments,
the first motion may be a right and left translational movement within a predetermined distance in the second direction,
the second motion may be a rolling movement according to rotation within a predetermined angular range with respect to the first axis in the first direction,
the third motion may be a vertical translational movement according to rising up and falling down within a predetermined vertical distance with respect to the third axis in the third direction, and
the fourth motion may be a pitching movement according to rotation within a predetermined angle with respect to the second axis in the second direction,
wherein the third motion and the fourth motion may be performed individually or in combination, regardless of the first motion and the second motion, so that the end-effector may be configured to perform any one motion from among the first motion to the fourth motion or a combined motion combining at least two motions from among the first motion to the fourth motion.

A method of controlling a surgical device, according to one or more embodiments, includes:
mounting an end-effector including a tool extending in a first direction on a mounting plate placed on a plane parallel to both a first axis in the first direction and a second axis in a second direction, the second direction crossing the first direction;
causing, by a first actuator including a first plate, the mounting plate on the first plate to perform a first motion in the second direction;
causing, by a second actuator, by rotating the first actuator with respect to the first axis in the first direction, a second motion of the end-effector on the mounting plate mounted on the first plate;
causing, by a third actuator, a third motion of the end effector, in combination with the first motion and the second motion of the end effector that are controlled by the first actuator and the second actuator, by vertically moving the first plate of the first actuator along a third axis in a third direction orthogonal to the first direction and the second direction; and
causing, by the third actuator, a fourth motion of the end effector, in combination with the first motion and the second motion of the end effector that are controlled by the first actuator and the second actuator, by rotating the first plate of the first actuator with respect to the second axis in the second direction.

In the method of controlling the surgical device, according to one or more embodiments,
a first motor provided in the first actuator may be configured to generate rotational power,
a first linear mover provided in the first actuator may perform a linear reciprocating movement through the rotational power, and
a translational lever provided in the first actuator may be operated by the linear reciprocating movement to cause a reciprocating movement of the mounting plate in the second direction, thereby causing the first motion of the end-effector.

In the method of controlling the surgical device, according to one or more embodiments,
a second motor of the second actuator may be configured to generate rotational power, and
a second linear mover of the second actuator may perform a linear reciprocating movement through the rotational power to control an inclination of the first plate on a second plate of the second actuator, thereby causing the second motion of the end-effector.

In the method of controlling the surgical device, according to one or more embodiments,
each of a third motor and a fourth motor provided in the third actuator, may be configured to generate rotational power, and
each of a third linear mover and a fourth linear mover, provided in the third actuator, may perform a linear reciprocating movement through the rotational power to make the first plate on a third plate provided in the third actuator rise up and fall down in the third direction, thereby causing any one of the third motion and the fourth motion of the end-effector.

In the method of controlling the surgical device, according to one or more embodiments,
the third motion may be performed according to operations of the third linear mover and the second fourth linear mover of a same distance, and
the fourth motion may be performed according to operations of the third linear mover and the second fourth linear mover of different distances.

### Brief Description of Drawings

FIG. 1 is a schematic perspective view of an end-effector type surgical device as a surgical device according to an embodiment of the present disclosure,
FIG. 2 is a schematic perspective view of a driver of a surgical device, from which an end-effector is removed, according to an embodiment of the present disclosure,
FIG. 3 is a schematic perspective view of a surgical device from which a housing is removed, according to an embodiment of the present disclosure,
FIG. 4 is an exploded partial perspective view of an end-effector of a surgical device and an actuator structure of a driver configured to drive the end-effector, according to an embodiment of the present disclosure,
FIG. 5 is a perspective view of an actuator structure of a surgical device, from which a housing and an end-effector are removed, according to an embodiment of the present disclosure,
FIG. 6 is a schematic partial perspective view of an actuator structure of a surgical device viewed in a different direction, according to an embodiment of the present disclosure,
FIG. 7 is a schematic partial perspective view of an actuator structure of a surgical device viewed in a different direction, according to an embodiment of the present disclosure,
FIG. 8 is a schematic partial perspective view of an actuator structure of a surgical device viewed directly from below, according to an embodiment of the present disclosure,
FIG. 9 illustrates each of a result of translational or shifting movement of a mounting plate of a surgical device and a posture or position of an end-effector arising therefrom, according to an embodiment of the present disclosure,
FIG. 10 illustrates a rising and falling operation, which is a third motion of a mounting plate in a surgical device, according to an embodiment of the present disclosure,
(A) and (B) of FIG. 11 illustrate vertical rising and falling or vertical translation, which is a third motion of an end-effector, and
(A) and (B) of FIG. 12 illustrate a pitching operation, which is a fourth motion of an end-effector.

### Mode for the Invention

Hereinafter, preferred embodiments of the present inventive concept are described in detail with reference to the accompanying drawings. However, the embodiments of the present inventive concept may be modified into various other forms, and the scope of the present inventive concept shall not be construed as being limited to the embodiments described below in detail. It is desirable that the embodiments of the present inventive concept be interpreted as being provided to more fully explain the present inventive concept to one of ordinary skill in the art. The same signs denote the same elements throughout. Furthermore, various elements and areas in the drawings are schematically drawn. Therefore, the present inventive concept is not limited by the relative sizes or distances illustrated in the accompanying drawings.

Terms such as first, second, etc. may be used to describe various components, but the components are not defined by these terms. The terms are used only for distinguishing one element from another element. For example, without deviating from the scope of the claims of the present inventive concept, a first element may be referred to as a second element, and vice versa.

The terms used in the present application are used only for describing particular embodiments and are not intended to limit the present inventive concept. A singular expression may include a plural expression, unless an apparently different meaning is indicated in the context. With respect to the present application, it will be further understood that the expressions "comprises" and "comprising" used herein specify the presence of stated features, integers, steps, operations, members, components, and/or groups thereof, but do not preclude the presence or addition of one or more other features, integers, operations, members, components, and/or groups thereof.

Unless otherwise defined, all of the terms used herein including technical terms and scientific terms have the same meaning as the meaning commonly understood by one of ordinary skill in the art. Also, the terms commonly used and having the meanings as defined in dictionaries shall be understood to have consistent meanings with the corresponding terms in the context of relevant arts, and unless explicitly defined so herein, the meanings of the terms shall not be understood to be excessively formal.

For directions described in this specifications, with respect to the directions used in the exemplary explanation about an operation of a surgical device according to the present disclosure, a first direction may be defined as being parallel to an X axis of a three-dimensional space having a 3-axis (X-Y-Z) coordinate system, a second direction may be defined as being parallel to a Y axis, and a third direction may be defined as being parallel to a Z axis, wherein the X axis may be referred to as a roll axis, the Y axis may be referred to as a pitch axis, and the Z axis may be referred to as a yaw axis.

Hereinafter, a surgical device and a method of the same are described according to one or more embodiments.

The surgical device according to the present disclosure may basically include the following components, which are a mounting plate on which an end-effector is mounted, and an actuator structure configured to move the end-effector on the mounting plate by a multi-degree of freedom, for example, four degrees of freedom.

The actuator structure may have a first actuator, a second actuator, and a third actuator which are interoperably connected to one another.

On the end-effector, a mechanical cutting tool, for example, a surgical saw for cutting a surgical site of a patient, for example, a bone, may be mounted. The end-effector may be arranged in the first direction, which is a reference direction, and thus, the surgical tool mounted on the end-effector may be oriented in the first direction.

The mounting plate, in a basic posture before operation, may be arranged on a first plane parallel to an X-Y plane in the three-dimensional space described above. A multi-degree of freedom actuator structure 100A described below may be attached below the mounting plate in a Z axis direction perpendicular to a normal line with respect to the X-Y plane.

With respect to a movement of the end-effector in such a surgical device according to the present disclosure, a first motion may be a right and left translational movement within a predetermined distance in the second direction, a second motion may be a rolling movement via rotation within a predetermined angular range with respect to a first axis in the first direction, a third motion may be a vertical translational movement via rising up and falling down within a predetermined vertical distance along a third axis in the third direction, and a fourth motion may be a pitching movement via rotation within a predetermined angle with respect to a second axis in the second direction, wherein the third motion and the fourth motion may occur individually or in a combined way, regardless of the first motion and the second motion. Thus, the end-effector may be configured to perform a singular motion from among the first to fourth motions or a combined motion in which at least two of the first to fourth motions are combined.

The first actuator, which is one element of the actuator structure, may be a component, on which the mounting plate is mounted, and may cause the mounting plate to perform a first motion M1 in the second direction parallel to the Y axis, for example, a right and left translational movement.

The second actuator, which is another element of the actuator structure, may rotate the mounting plate together with the first actuator within a predetermined (certain) angular range with respect to a rolling axis (the first axis) in the second direction and may cause the mounting plate to perform a second motion M2, for example, a rolling movement M2.

The third actuator, which is another element of the actuator structure, may cause the mounting plate together with the second actuator to perform a third motion M3, which is a top and bottom translational movement of rising and falling by a certain distance in the Z direction, for example, a top and bottom translational movement M3, and at the same time, may rotate the mounting plate together with the second actuator within a certain angular range with respect to the pitching axis (the second axis) and may cause a fourth motion M4 of the mounting plate, for example, a pitching movement M4.

The actuators may have power transmission units, that is, the power transmission units as a powertrain system transmitting rotational power from motors as rotational power or linear power.

The first actuator may include: a first motor configured to drive the mounting plate on a first plate; and a first power transmission unit configured to transmit power of the first motor to operate the mounting plate by using the first motor.

The first power transmission unit may include: a first linear mover configured to convert a rotary motion of the first motor into a linear reciprocating motion; and a translational lever configured to transfer the linear motion of the first linear mover to the mounting plate on the first plate to cause a translational motion of the mounting plate.

The linear motion from the first linear mover may occur in a direction across the plane of the mounting plate, and the translational lever may be hinge-coupled to the mounting plate and may convert the linear motion from the first linear mover into the direction of the translational motion of the mounting plate.

The second actuator may include: a second plate rotatably hinge-coupled to the first plate; a second motor mount integrally coupled to the second plate; a second motor mounted on the second motor mount; a second linear mover configured to convert rotational motion of the second motor into a linear reciprocating motion; and a rolling lever having both ends connected to the second linear mover and the first plate and configured to cause the first plate to perform the second motion with respect to the second plate.

The first to third actuators may be arranged in a housing configured to protect the first to third actuators, and the third actuator may include: a third plate, the position of which is fixed with respect to the housing; a third motor and a fourth motor configured to cause the third motion and the fourth motion of the first plate; a third motor mount configured to support the third motor and the fourth motor and integrally fixed to the third plate; a third linear mover and a fourth linear mover respectively connected to the third motor and the fourth motor and configured to convert rotary motions into linear reciprocating motions; and a third operating rod and a fourth operating rod respectively connected to the third linear mover and the fourth linear motor and configured to collaboratively cause the third motion and/or the fourth motion of the first plate.

The third operating rod may be directly hinge-coupled to the first plate, and the fourth operating rod may be hinge-coupled to the first plate through a link lever.

The first to third actuators may be arranged in a housing configured to protect the first to third actuators, and the third actuator may include: a third plate, the position of which is fixed with respect to the housing; a third motor and a fourth motor configured to cause the third motion and the fourth motion of the first plate; a third motor mount configured to support the third motor and the fourth motor and integrally fixed to the third plate; a third linear mover and a fourth linear mover respectively connected to the third motor and the fourth motor and configured to convert rotary motions into linear reciprocating motions; and a third operating rod and a fourth operating rod respectively connected to the third linear mover and the fourth linear motor and configured to collaboratively cause the third motion and/or the fourth motion of the first plate.

The third operating rod may be directly hinge-coupled to the first plate, and the fourth operating rod may be hinge-coupled to the first plate through a link lever.

These structures can be clearly understood with the corresponding descriptions given with reference to the drawings.

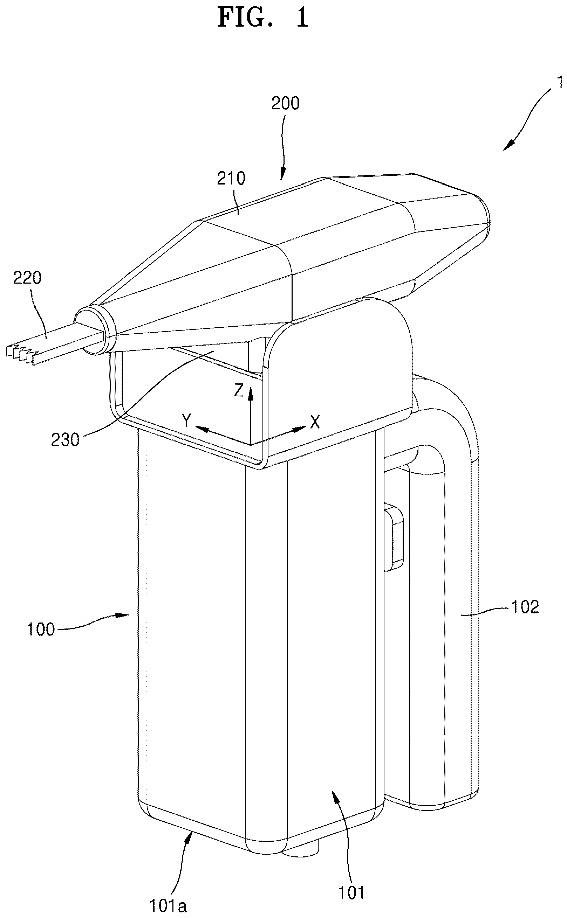

FIG. 1 is a schematic perspective view of an end-effector type surgical device according to an embodiment of the present disclosure.

Referring to FIG. 1, the end-effector type surgical device (hereinafter, the surgical device) 1 may include an end-effector 200 and an end-effector driver (hereinafter, a driver) 100, on which the end-effector 200 is mounted.

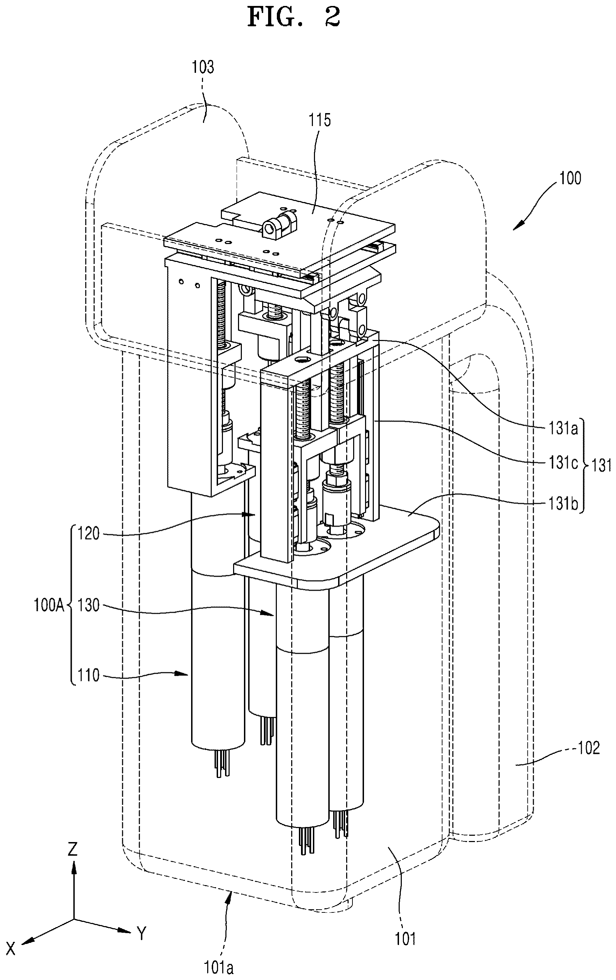

The driver 100 may include a housing 101 providing a grip 102, a mounting plate 112 (see FIG. 2), which is exposed above the housing 101, and on which the end-effector 200 is mounted, and a protection fence at both sides 103 configured to protect the mounting plate 115.

The end-effector 200 may include a surgical tool 220, such as a saw or a drill, and a case 210 including a driving device configured to drive the surgical tool 220, for example, a driving motor, wherein at a side of the case 210, that is, below the case 210, according to the present embodiment, a mounting foot 230 coupled to the mounting plate 115 (see FIG. 2) may be provided. The coupling of the mounting foot 230 to the mounting plate 115 (see FIG. 2) may be realized by a detachable fixation-type coupling structure or fastening structure.

FIG. 2 is a schematic perspective view of the driver 100, from which the end-effector 200 is removed, in the surgical device illustrated in FIG. 1. In FIG. 2, the housing 101 and the grip 102 are illustrated by dotted lines to show the actuator structure 100A inside the housing 101 of the driver 100.

The housing, at one side of which the grip 102 is formed, may have a box shape having an open upper portion corresponding to the end-effector 200. The actuator structure 100A configured to operate the mounting plate 115 may be fixed in the housing 101.

The actuator structure 100A may include a first actuator 110, a second actuator 120, and a third actuator 130 for controlling a posture and a position of the mounting plate 115 by a multi-degree of freedom, for example, the maximum 4 degrees of freedom.

The actuator structure 100A may be solidly fixed to an inner wall of the housing 101 by a third bracket 131 of the third actuator 130.

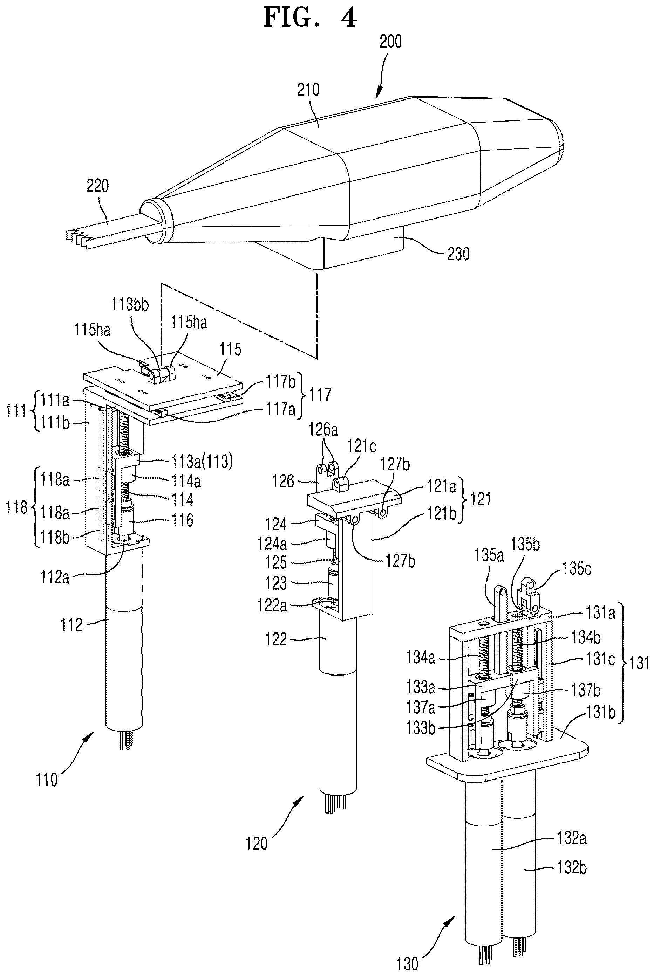

The third bracket 131 may be a base structure fixing the actuator structure 100A in the housing 101 and may have a function of a main frame generally supporting the actuator structure 100A. Thus, a motion of the end-effector may be a relative motion with respect to the third bracket 131. Though it will be described in detail below, the third bracket 131 may include a third plate 131a, a third motor mount 131b, and a support pillar 131c integrally connecting the third plate 131a with the third motor mount 131b, which are to be described below in more detail with reference to FIG. 4.

Referring to FIG. 1 again together with FIG. 2, in the surgical device 1 having the example structure as described above, the end-effector 200 may, while being mounted on the driver 100, through the driver 100, be capable of various motions by three degrees of freedom or four degrees of freedom in a 3-axis (X-Y-Z) direction, that is, an X axis in a first direction, a Y axis in a second direction intersecting or crossing the first direction, and a Z axis in a third direction intersecting or crossing the first direction and the second direction.

FIGS. 1 and 2 illustrate a state in which the driver 100 sustains the end-effector 200 as an initial neutral posture. An X-Y plane in the X-Y-Z coordinate system may be parallel to a plane on which the bottom of the driver 100, that is, a floor 102a of the housing 101, is placed, and may also be parallel to a plane of the mounting plate 115, on which the mounting foot 230 of the end-effector 200 is mounted. The Z axis orthogonal to this plane may be parallel to a direction in which the housing 101 of the driver is uprightly arranged and may also be parallel to an extension direction of the surgical tool 220 of the end-effector 200.

In a three-dimensional space by the X-Y-Z three axes, an operating center of the mounting plate 115 may be position-traceably flexible in the X-Y-Z three-dimensional space with respect to the motion of the actuator structure 100A. Thus, it may be possible to control various postures and positions of the surgical tool 220 of the surgical device 1 while the driver 100 has a fixed position.

As described above, the movement operation, that is, the motion, of the end-effector 200 within the X-Y-Z coordinate system is described, to help understand each of various motions according to the present disclosure or a combined motion in which these motions are combined.

According to various operation motions of the actuator structure 100A described above, while the driver 100 including the actuator structure 100A is placed at a limited position in a surgical space or at a fixed position due to a restricted posture of a surgeon, the end-effector 200 on which the surgical tool 220 is mounted is capable of various motion operations in a 3-axis coordinate system.

To describe the motions of the end-effector 200, part or all of the terms used in definitions of the motions of an airplane or a vehicle may be used. Various operational motions of the end-effector 200 may include the motions described above.

The motions may include the first motion M1, which is a right and left translational movement in the second direction along the Y axis, according to the first actuator 110, the second motion M2, which is a rolling motion with respect to a roll axis in the first direction along the X axis, according to the second actuator 120, the third motion M3, which is a top and bottom translational movement of rising and falling by a certain distance in the third direction along the Z axis, according to the third actuator 130, and at the same time, the fourth motion M4, which is a pitching motion with respect to a pitching axis in the second direction, wherein at least one of these motions may be performed or two or more motions may be performed in combination.

The translational operation denotes a change of a position of the end-effector in a right and left direction along the Y axis without a posture change in a longitudinal axial direction and in a top and bottom direction along the Z axis, according to the second motion and the third motion.

The movements in a X direction, a Y direction, and a Z direction may correspond to or be in parallel with the X, Y, and Z axes, respectively, and may also refer to "slightly oblique movements having directionalities" of the X, Y, and Z axes.

Various types of these motions may be performed in a combined way, according to the present disclosure. Various types of these motions may occur through the driver 100, which will be described in detail below.

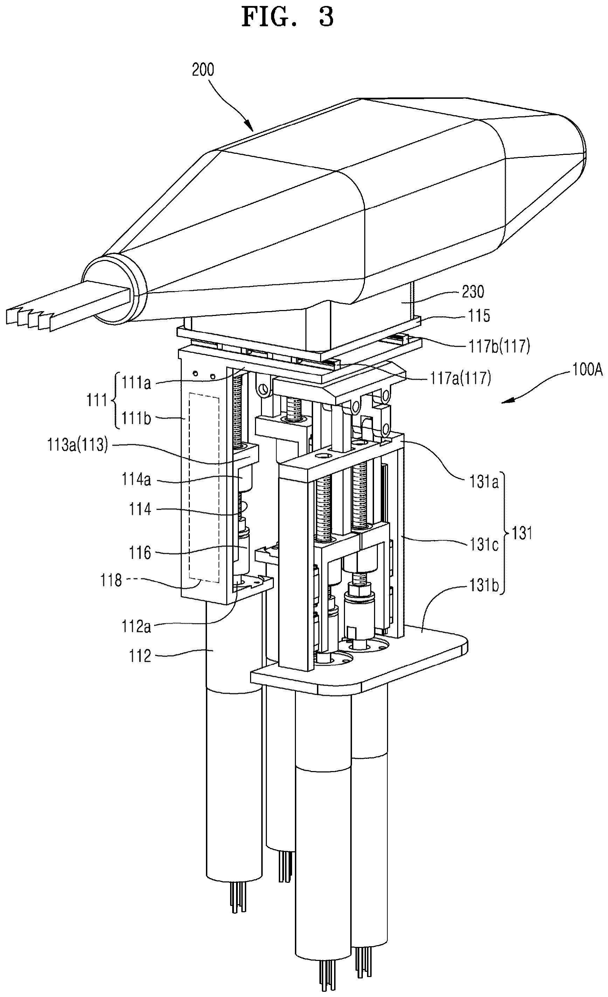

FIG. 3 is a schematic perspective view of the surgical device 1 from which the housing 102 (see FIGS. 1 and 2) is removed, and FIG. 4 is an exploded partial perspective view of the end-effector 200 and the actuator structure 100A of the driver 100 driving the end-effector 200.

Referring to FIG. 3, the end-effector 200 may be coupled to the mounting plate 115 of the driver 100 by the mounting foot 230 at a lower portion of the end- effector 200, and the mounting plate 115 may be coupled to the actuator structure 100A.

The mounting plate 115 moved by the actuator structure 100A may ultimately cause all motions of the end-effector 200, and all the motions may occur as relative movements with respect to the third bracket 131, the third plate 131a, or the third motor mount 131b of the third actuator 130. The third plate 131a may be, together with the third motor mount 131b, included in the third bracket 131, which is the main frame for sustaining the entire structure of the third actuator 130, and the position of the third plate 131a may be fixed with respect to the housing 101 of the driver 100.

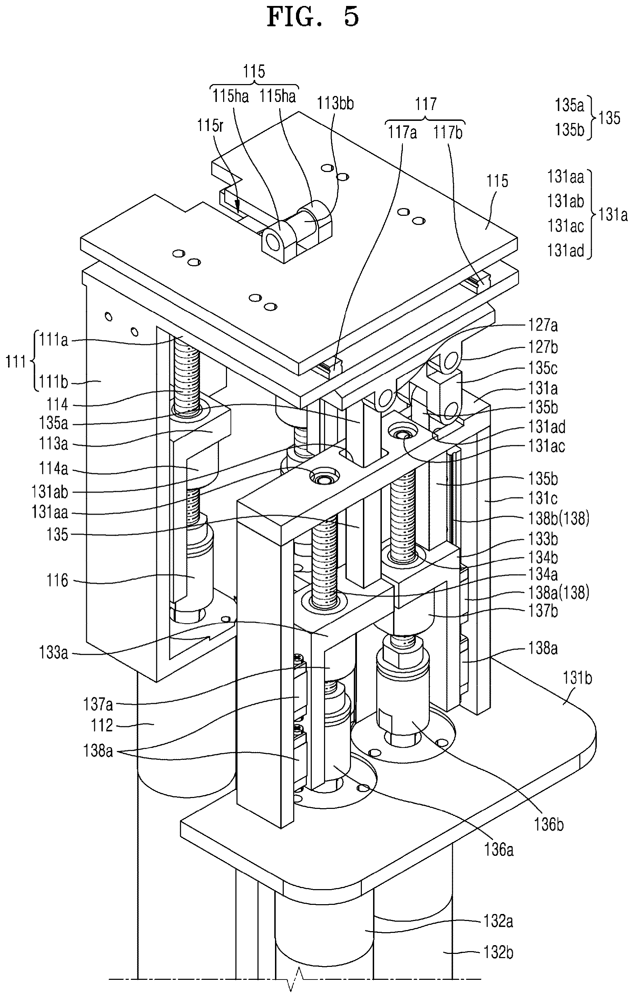

Hereinafter, FIGS. 3 and 4 are mainly referred to for describing the actuator structure 100A. Although FIGS. 5 to 8 may be partially referred to for helping understanding, FIGS. 5 to 8 are to be separately described again.

Referring to FIGS. 3 and 4 together, the actuator structure 100A configured to operate the end-effector 200 may be a coupling structure in which the first actuator 110, the second actuator 120, and the third actuator 130 are interoperably coupled to one another.

### <Mounting plate>

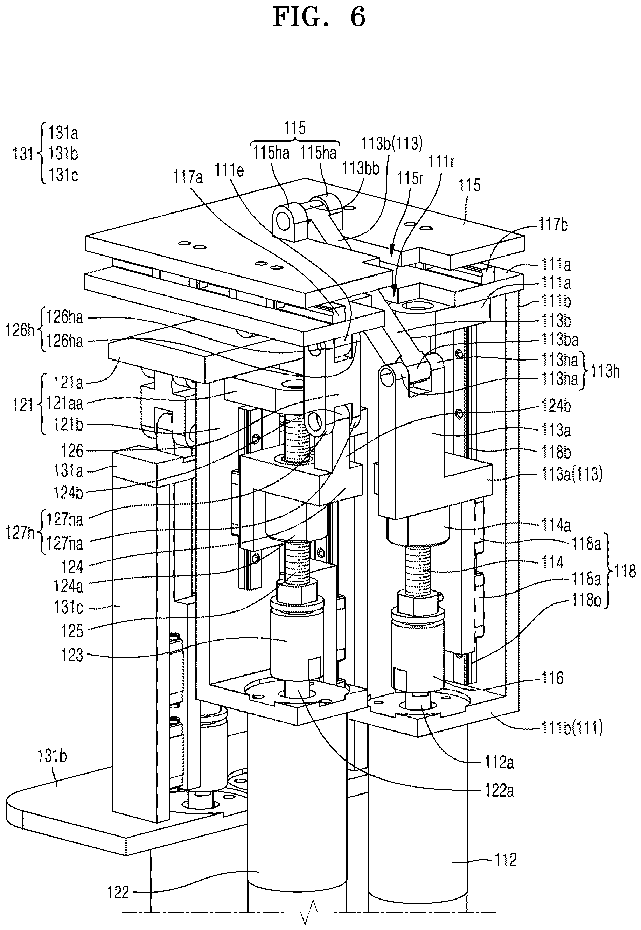

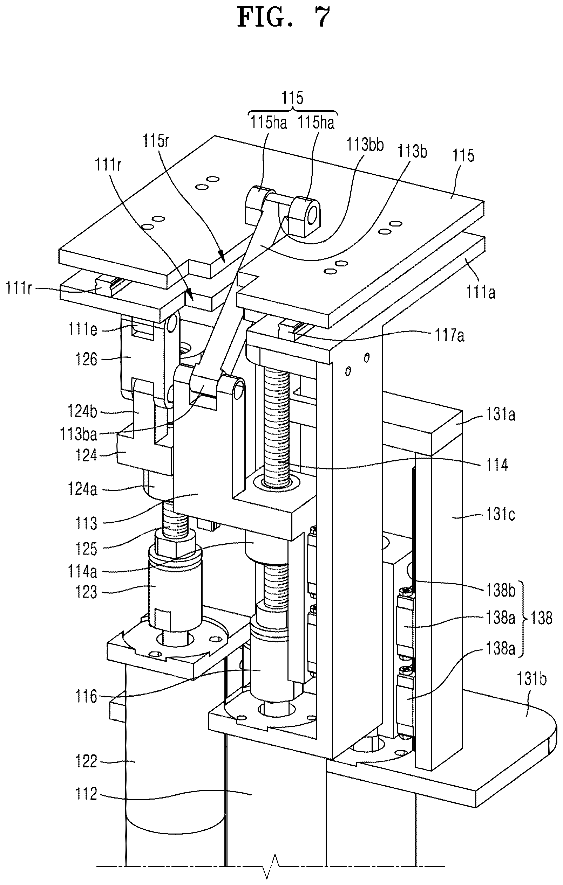

The mounting plate 115 may be mounted on a first plate 111a of the first actuator 110 to be capable of performing a reciprocating movement by a certain distance through a linear guide rail device 117 therebelow. For this reciprocating movement of the mounting plate 115, a third hinge portion 115h, for example, a pair of pivot protrusions 115ha, as illustrated, may be provided, and a pivot end 113bb at one side (an upper end in the drawing) of a translational lever 113b (see FIGS. 6 and 7) described below may be inserted into and mutually rotatably coupled to the third hinge portion 115h, and thus, through a rocking motion combining rotational movement and linear movement of the translational lever 113b, the mounting plate 115 may perform the reciprocating movement on the first plate 111a, thereby performing a linear translational movement in the second direction. An operation lever structure 113 (see FIGS. 6 and 7) described below may include the translational lever 113b and a first linear mover 113a.

### <First Actuator>

The first actuator 110 may include a first bracket 111 and a first motor 112, wherein the first bracket 111 may support the mounting plate 115 to be capable of a right and left translational or shift movement within an operation range of a pre-set working distance through the linear guide rail device 117 including two slide guide rails 117a and 117b, and the first motor 112 may be mounted on the first bracket 111 and may supply power for the translational movement of the mounting plate 115. The first bracket 111 may include the first plate 111a and a first motor mount 111b, formed of a plurality of integrally assembled components or formed as a single structure.

A rotational shaft 112a of the first motor 112 mounted on the first motor mount 111b and causing the right and left shift (translational) movement of the mounting plate 115 may be connected to a first linear transport screw 114 through a first coupler 116, and the first linear transport screw 114 may be screw-fixed to a first female screw portion 114a integrally coupled to the first linear mover 113a moving the operation lever structure and may allow the first linear mover 113a to perform a reciprocating movement along a Z axis within the operation range. Here, the first motor mount 111b may approximately have an "L" shape, the first motor 112 may be mounted below the first motor mount 111b, and, above the first motor 112, between portions in a Z direction parallel to the first linear mover 113a, a guide rail structure 118 including a guide rail 118a and a slider 118b instructing a linear reciprocating movement of the first linear mover 113a in an extension direction of the first linear transport screw 114 may be mounted. This aspect will be clearly understood with reference to the descriptions of FIGS. 6 and 7.

### <Second Actuator>

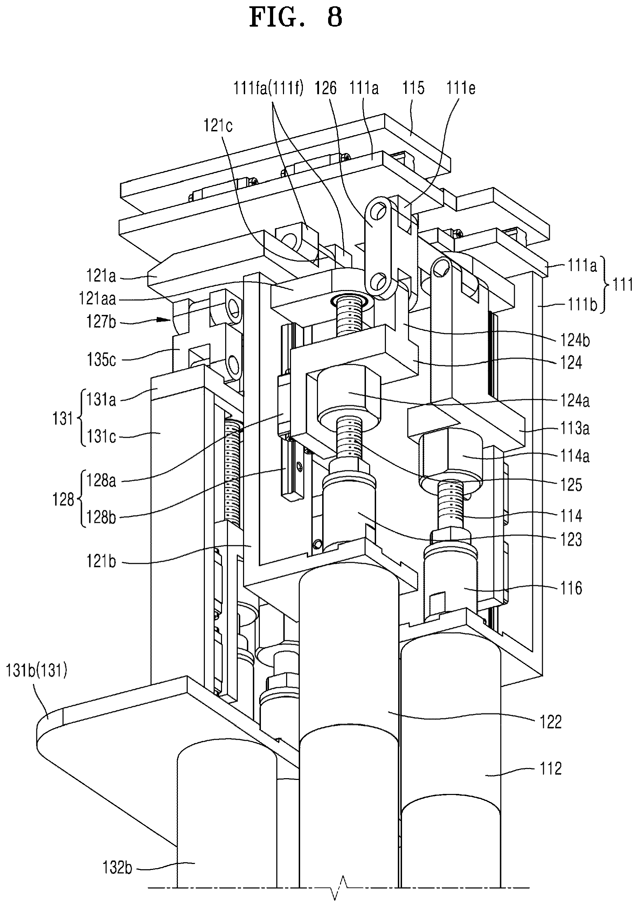

The second actuator 120 may include a second motor 122 causing a rolling motion M2 of the mounting plate 115 and a second bracket 121 supporting the second motor 122. In detail, the second actuator 120 may include a second female screw portion 124a performing a reciprocating movement along with a second linear transport screw 125 in a direction of the Z axis according to rotation of the second motor 122, a second linear mover 124 to which the second female screw portion 124a is integrally coupled, and a rolling lever 126 pivotably coupled to the second linear mover 124 and a pivot protrusion 111e (see FIG. 8) of the first plate.

A hinge portion 126h at one side and a hinge portion 127h at the other side may be provided at both ends of the rolling lever 126, and a pivot end 124b pivotably coupled to the hinge portion 127h at the other side of the rolling lever 126, may be formed at a side (an upper end in the drawing) of the second linear mover 124.

A pair of pivot ends 126ha may be formed at an end (an upper end in the drawing) of the rolling lever 126 and may be pivotably coupled to a pivot protrusion 111e (see FIG. 8) provided at a lower surface of the first plate 111a. Also, a protrusion-type pivot end 121c may be formed at a side of an upper surface of a second plate 121a and may be inserted into and pivotably coupled to a first hinge portion 111f having a pair of hinge protrusions 111fa formed at a lower surface of the first plate 111a.

According to this structure, the second actuator 120 may be interoperably coupled to two points on the lower surface of the first plate 111a, through the pivot end 121c of the second plate 121a and the pivot end 124b of the second linear mover 124. Thus, during operation of the second actuator 120, while the first plate 111a is being hinge-coupled to the second plate 121a via the pivot end 121c, as the rolling lever 126, which is pivotably coupled to the pivot protrusion 111e with respect to the first plate 111a, rises and falls through the second linear mover 123a, the first plate 111a may perform an inclination operation, and thus, a rolling motion of the mounting plate 115 may be performed.

The second bracket 121 may include a second motor mount 121b on which the second motor 122 is mounted and the second plate 121a. The second motor mount 121b may be integrally coupled to the second plate 121a, and according to another embodiment, the second bracket 121 may be integrally formed with the second plate 121a as a single body.

### <Third Actuator>

As described above, the third actuator 130 may be configured to generally support the actuator structure 100A described above, may include the third bracket 131 for the supporting, and may include a third motor 132a and a fourth motor 132b, the two motors for a pitching motion and a bottom and top rising and falling movement performed with respect to the first plate 111a indirectly through the second plate 121a.

Here, the third bracket 131 may include the third motor mount 131b and the third plate 131a supporting the third motor 132a and the fourth motor 132b in parallel. Here, the third plate 131a may support rotation of a third linear transport screw 134a and a fourth linear transport screw 134b described below.

The third actuator 130 may be operably connected to the first plate 111a of the first bracket 111 by an operating rod structure 135 operated by each of the third motor 132a and the fourth motor 132b.

In detail, the operating rod structure 135 may include a third operating rod 135a operably connected to the third motor 132a and a fourth operating rod 135b operably connected to the fourth motor 132b.

Ends (lower ends in the drawing) of the third operating rod 135a and the fourth operating rod 135b may be respectively coupled to a third linear mover 133a and a fourth linear mover 133b performing linear reciprocating motions through the third linear transport screw 134a and the fourth linear transport screw 134b respectively connected to the third motor 132a and the fourth motor 132b within an operation range of a working distance pre-set by the third motor 132a and the fourth motor 132b. A third coupler 136a and a fourth coupler 136b as described above may be coupled to rotational shafts of the third motor 132a and the fourth motor 132b, respectively.

The other ends (upper ends in the drawing) of the third operating rod 135a and the fourth operating rod 135b may be coupled to be pivotable, that is, hinge-coupled to be relatively rotatable, with respect to the second plate 121a. Here, the fourth operating rod 135b may be pivotably connected, through a link lever 135c, to a pivot protrusion 127ha at one side and a pivot protrusion 127hb at the other side provided at the second plate 121a.

Thus, the third actuator 130 may generate a pitching motion and/or an top and bottom rising and falling motion, regardless of the presence of a motion by the first actuator 110 and/or a motion by the second actuator 120, that is, a right and left translational movement by the first actuator 110 and/or a rolling motion by the second actuator 120.

Thus, when the motion by the first actuator 110 and/or the motion by the second actuator 120 are/is present, the pitching motion and/or the top and bottom rising and falling motion may be generated in addition to these motions, and when there is not the motion by any of the first actuator 110 and the second actuator 120, the pitching motion and/or the rising and falling motion of the mounting plate 115 may be generated.

FIG. 5 is a perspective view of a portion of an actuator structure when a housing and an end-effector are removed.

Referring to FIG. 5, as described above, the third actuator 130 may include two motors, that is, the third motor 132a and the fourth motor 132b, and the third bracket 131 supporting these motors.

The third bracket 131 may include the third motor mount 131b supporting the third motor 132a and the fourth motor 132b in parallel, and the third plate 131a integrally coupled to the third motor mount 131b.

The third coupler 136a and the fourth coupler 136b described above may be coupled to the rotational shafts of the third motor 132a and the fourth motor 132b, respectively, and the third linear transport screw 134a and the fourth linear transport screw 134b may be coupled to the third coupler 136a and the fourth coupler 136b, respectively.

The third linear mover 133a and the fourth linear mover 133b each performing a reciprocating motion within an operation range of a working distance pre-set by the third motor 132a or the fourth motor 132b may be coupled to the third linear transport screw 134a and the fourth linear transport screw 134b, respectively.

A third female screw portion 137a and a fourth female screw portion 137b respectively screw-coupled to the third linear transport screw 134a and the fourth linear transport screw 134b may be integrally fixed to the third linear mover 133a and the fourth linear mover 133b, respectively.

The third linear mover 133a and the fourth linear mover 133b may be connected to the operating rod structure 135. In detail, the third linear mover 133a and the fourth linear mover 133b may be respectively and integrally coupled to the third operating rod 135a and the fourth operating rod 135b of the operating rod structure 135 that are respectively arranged to be parallel to the third linear transport screw 134a and the fourth linear transport screw 134b.

A guide rail structure 138 including a guide rail 138a and a slider 138b may be mounted between the third linear mover 133a and the fourth linear mover 133b and both support pillars 131c facing the third linear mover 133a and the fourth linear mover 133b, and thus, linear movements of the third linear mover 133a and the fourth linear mover 133b through the linear transport screw 134a and the fourth linear transport screw 134b may be stably supported.

Through holes 131aa and 131ac supporting the rotation of the third linear transport screw 134a and the fourth linear transport screw 134b may be formed in the third plate 131a, and a bearing may be provided in each of the through holes 131aa and 131ac to rotatably support an axial end of each of the third linear transport screw 134a and the fourth linear transport screw 134b. Also, through holes 131ab and 131ad through which the third operating rod 135a and the fourth operating rod 135b pass, the third operating rod 135a and the fourth operating road 135b being respectively and integrally coupled to the third linear mover 133a and the fourth linear mover 133b that are moving, may be formed in the third plate 131a.

The operating rod structure 135 may include the third operating rod 135a operably connected to the third motor 132a and the fourth operating rod 135b operably connected to the fourth motor 132b.

As described above, the other ends (upper ends in the drawing) of the third operating rod 135a and the fourth operating rod 135b may be coupled to be pivotable, that is, hinge-coupled to be relatively rotatable, with respect to the first plate 111a.

In detail, the upper end of the third operating rod 135a may be hinge-coupled to be pivotable with respect to the pivot protrusion 111e provided in the first plate 111a. Here, the upper end of the fourth operating rod 135b may be pivotably connected, through the link lever 135c, to the first hinge portion 111f having the pair of hinge protrusions 111fa provided in the first plate 111a. Here, the link lever 135c may be configured to transfer a force by the fourth operating rod 135b to the first plate 111a through the first hinge portion 111f while permitting a mutual misalignment or offset between an end of the fourth operating rod 135b and the first hinge portion 111f due to an inclination operation of the first plate 111a.

According to this pivoting structure of the third operating rod 135a and the fourth operating rod 135b, the second plate may be allowed to rise and fall according to simultaneous reciprocating movements of the third operating rod 135a and the fourth operating rod 135b, and thus, both of the first actuator 110 and the second actuator 120 may be allowed to perform a rising and falling movement in a top and bottom direction (a Z direction) or a top and bottom translation movement, without their postures affected.

Also, when the third operating rod 135a and the fourth operating rod 135b differently operate from each other, for example, when the fourth operating rod 135b performs a reciprocating motion of a relatively longer distance than the third operating rod 135a, or when the fourth operating rod 135b performs the reciprocating motion while transportation of the third operating rod 135a is being stopped, the first plate 111a may perform a pitching operation with respect to the pivot protrusion 111e. That is, only one of the pitching operation and the rising and falling operation of the third actuator 130 may be generated, and the both movements may be performed as a combined operation of pitching-rising and falling operations. Also, after the third actuator 130 rises to a target position, the third actuator 130 may perform the pitching operation by a target pitching angle and may maintain the same state.

FIGS. 6 and 7 are schematic partial perspective views of the actuator structure 100A in two directions that are different from the direction in FIG. 5 and show a schematic structure of the first actuator 110 and the second actuator 120 in detail in different directions.

FIG. 6 and FIG. 7 differ in angles, whereby FIG. 6 focuses on the second actuator 120, and FIG. 7 focuses on the first actuator 110.

Referring to FIGS. 6 and 7, in overall, the linear guide rail device 117 including the two slide guide rails 117a and 117b may be mounted on the first plate 111a, and the mounting plate 115 may be mounted thereon to be capable of a reciprocating movement in a planar direction.

The structure and operation of the first actuator 110 configured to generate the translational motion M1, that is, the translational movement of the mounting plate 115, are described below.

The mounting plate 115 may be configured to be moved by a translational structure 113 including the translational lever 113b directly and pivotably connected to the mounting plate 115. The translational structure 113 may include the first linear mover 113a performing a reciprocating motion through the first linear transport screw 114, and the rotational translational lever 113b having both ends hinge-coupled to the first linear mover 113a and the mounting plate 115.

A first recessed portion 115r and a second recessed portion 111r for providing a space needed to arrange and operate the translational lever 113b may be formed on the mounting plate 115 and the first plate 111a therebelow.

A detailed structure of the translational structure 113 is described below. The pivot end 113bb at the one side of the translational lever 113b may be pivotably coupled to the third hinge portion 115h provided on the mounting plate 115, and a pivot end 113ba at the other side of the translational lever 113b may also be pivotably coupled to a second hinge portion 113h having two hinge protrusions 113ha protruding from an end of the first linear mover 113a.

The pivot end 113bb at the one side and the pivot end 113ba at the other side may be provided at both ends of the translational lever 113b, and the pivot end 113ba at the one side and the pivot end 113bb at the other side may be complementarily inserted into and coupled to the second hinge portion 113h and the third hinge portion 115h provided in the mounting plate 115, respectively. Thus, the translational lever 113b may be pivotable with respect to the first linear mover 113a and the mounting plate 115 through the second hinge portion 113h of the first linear mover 113a and the third hinge portion 115h of the mounting plate 115, respectively.

According to the structure above, the rotation of the first motor 112 transferred by the first coupler 116 may cause a linear movement of the first linear mover 113a, and the translational lever 113b pivotably connected thereto may rock in an appropriate posture to cause the mounting plate 115 to perform a movement in a Y axis direction, that is, a right and left translation or shifting operation.

A mounting structure of the second actuator 120 generating the rolling motion M2 is described below further.

The second bracket 121 of the second actuator 120 may include the "L"-shaped second motor mount 121b below the second plate 121a. A shaft support portion 121aa rotatably supporting an end (an upper end in the drawing) of the second linear transport screw 125 may be fixed to a side (a right side in the drawing) of the second plate 121a. A bearing rotatably supporting the end of the second transport screw 125 may be mounted on the shaft support portion 121aa.

The pivot end 124b described above may be formed at a side (an upper side in the drawing) of the second linear mover 124 performing a linear reciprocating motion through the second linear transport screw 125 rotated by the second motor 122, and the hinge portion 127h at a side (a lower side in the drawing) of the rolling lever 126 may be rotatably coupled to the pivot end 124b.

According to this structure, the second actuator 120 may be interoperably coupled to two points of the lower surface of the first plate 111a through the pivot end 121c of the second plate 121a and the pivot end 124b of the second linear mover 124.

Thus, during operation of the second actuator 120, while the first plate 111a is being hinge-coupled to the second plate 121a through the pivot end 121c, as the rolling lever 126, which is pivotably coupled to the pivot protrusion 111e with respect to the first plate 111a, rises and falls through the second linear mover 123a, the first plate 111a may perform an inclination operation, and thus, a rolling motion of the mounting plate 115 may be performed.

The second bracket 121 may include the second motor mount 121b on which the second motor 122 is mounted and the second plate 121a. The second motor mount 121b may be integrally coupled to the second plate 121a, and according to another embodiment, the second bracket 121 may be integrally formed with the second plate 121a as a single body.

FIG. 8 is a perspective view, shown from below, of the entire mounting structure of the second actuator 120.

As illustrated in FIG. 8, the pivot protrusion 111e to which the rolling lever 126 described above is hinge (pin)-coupled and the first hinge portion 111f to which the pivot end 121c of the second plate 121a is pin-coupled may be formed on the lower surface of the first plate 111a.

A guide rail structure 128 may be mounted between parallel portions of the second linear mover 124 and the "L"-shaped second motor mount 121b. The guide rail structure 128 may include a guide rail 128b mounted on a vertical partial inner wall of the second motor mount 121b and a slider 128a moving along the guide rail 128b and fixed to an inner surface of a vertical portion of the second linear mover 124.

The guide rail structure 128 may support a stable relative movement of the second linear mover 124 with respect to the second motor mount 121b.

According to this structure, the second actuator 120 may be interoperably coupled to two points of the lower surface of the first plate 111a through the pivot end 121c of the second plate 121a and the pivot end 124b of the second linear mover 124. Thus, during operation of the second actuator 120, while the first plate 111a is being hinge-coupled to the second plate 121a through the pivot end 121c, as the rolling lever 126, which is pivotably coupled to the pivot protrusion 111e with respect to the first plate 111a, rises and falls through the second linear mover 123a, the first plate 111a may perform an inclination operation, and thus, a rolling motion of the mounting plate 115 may be performed.

Hereinafter, various operations according to a multi-degree of freedom of the surgical driver 100 according to the present disclosure are described.

The actuator structure 100A of the surgical driver 100 according to the present disclosure may have the three actuators, operations of the actuators may be individually performed, and the operations of the actuators may be simultaneously performed to generate highly diverse and combined end-effector movements. Hereinafter, the operations are described with reference to the drawings.

### Operation of the First Actuator

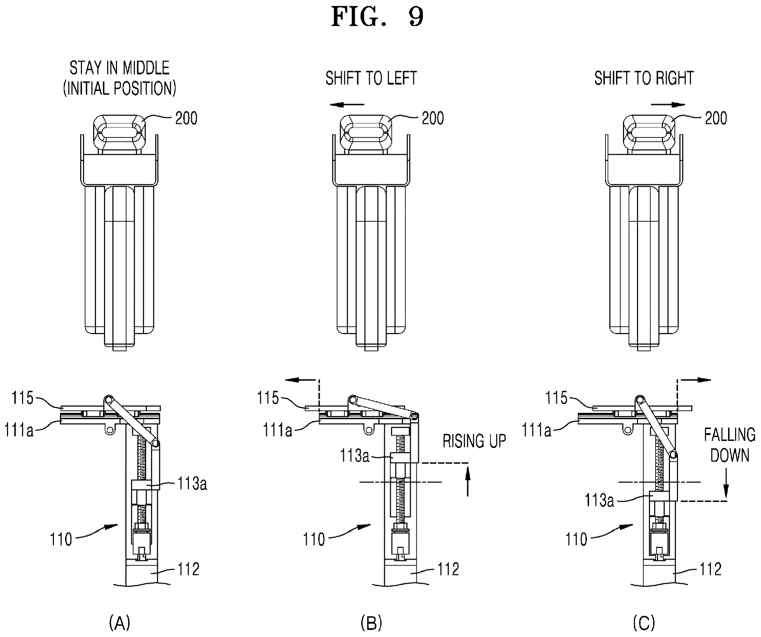

(A), (B), and (C) of FIG. 9 each shows a result of a translational movement or shifting operation, which is the first motion M1 of the mounting plate 115 through the first actuator 110, and a posture or a position of the end-effector 200 arising therefrom.

In FIG. 9, (A) illustrates a state in which the first actuator 110 stays in a middle position (initial position), (B) illustrates a state in which the first motor 112 of the first actuator 110 operates so that the first linear mover 113a moves upwards above the initial middle position (the middle position of an operating area), and (C) illustrates a state in which the first actuator 110 moves downwards below the middle position.

Thus, in FIG. 9, in the state of (A), the end-effector 200 may stay in a middle position according to the middle position (the initial position) of the first actuator 110, in the state of (B), due to rising up of the first linear mover 113a, the operational lever-type translational lever 113b may operate to push the mounting plate 115, and thus, the end-effector 200 thereabove may be shifted to the left side, and in the state of (C), the first actuator 110 may fall down below the middle position to pull the mounting plate 115, and thus, the end-effector 200 thereabove may be shifted to the right side.

### Operation of the Second Actuator

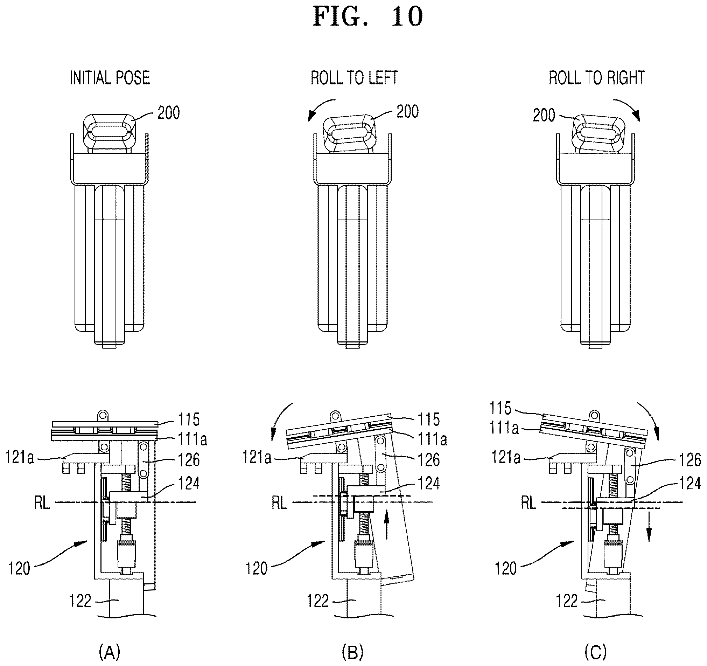

(A), (B), and (C) of FIG. 10 each shows a result of the second motion M2, that is, a rolling operation, of the first plate 111a through the second actuator 120, and a posture of the end-effector 200 arising therefrom.

In FIG. 10, (A) illustrates a state in which the second actuator 120 stays at a middle position (an initial posture), (B) illustrates a state in which the second motor 122 of the second actuator 120 operates so that the second linear mover 124 rises up, and the first plate 111a is rolled to the left side, that is, inclined in one direction (a counter clockwise direction in the drawing) by a certain angle, and (C) illustrates a state in which the second motor 122 of the second actuator 120 operates so that the second linear mover 124 falls down below a reference position, and the first plate 111a is rolled to the right side, that is, inclined in one direction (a clockwise direction in the drawing) by a certain angle.

Thus, in FIG. 10, in the state of (A), the end-effector 200 may stay in the middle position toward the front side in the drawing according to the middle position (the initial posture) of the second actuator 120, in the state of (B), the end-effector 200 may be rolled to the left side by a certain angle, and in the state of (C), the end-effector 200 may be rolled to the right side by a certain angle.

### Operation of Third Actuator

The third actuator 130 may perform the third motion M3, which is the vertical translational movement, and the fourth motion M4, which is the pitching movement, separately or in combination.

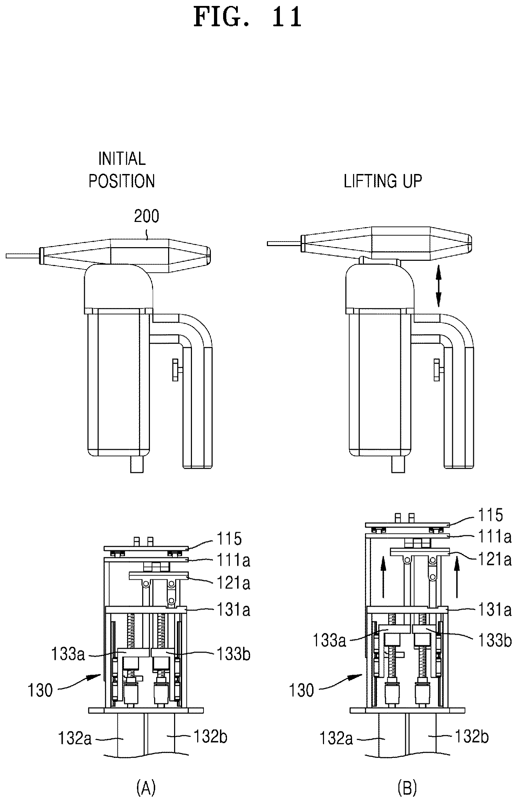

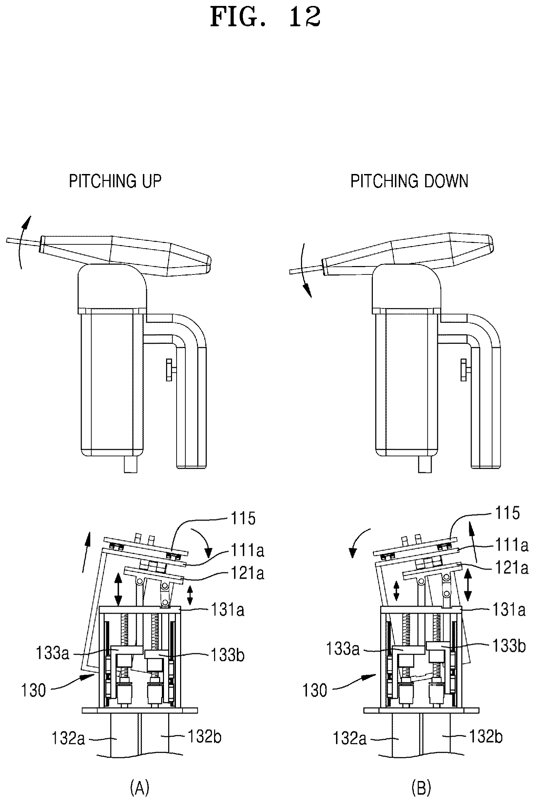

(A) and (B) of FIG. 11 illustrate the vertical rising and falling or vertical translation operation, which is the third motion M3 of the end-effector 200, and (A) and (B) of FIG. 12 illustrate the pitching operation, which is the fourth motion M4 of the end-effector 200.

As illustrated in (A) of FIG. 11, through the third actuator 130 in an initial state or posture, the end-effector 200 may also maintain an initial state or posture. In this state, when the third linear mover 133a and the fourth linear mover 133b of the third actuator 130 simultaneously operate and simultaneously rise up by the same distance, the end-effector may vertically rise up from the initial state or may vertically rise up and then fall down, as illustrated in (B).

The third motion M3, which is the vertical translation, may be realized by the synchronized movements of the third linear mover 133a and the fourth linear mover 133a, which simultaneously move by the same distance.

Also, in the state of (A) of FIG. 11, when the third linear mover 133a and the fourth linear mover 133b of the third actuator 130 move by different distances or in different forms from each other, a pitching up or pitching down operation with the posture illustrated in (A) or (B) of FIG. 12 may be performed.

That is, in FIG. 11, (A) shows a state in which the third motor 132a and the fourth motor 132b differentially operate, and the third linear mover 133a rises up by a relatively greater height than the fourth linear mover 133b, so that the end-effector 200 is lifted at the front side, and on the contrary, (B) shows a state in which the third motor 132a and the fourth motor 132b differently operate, and the fourth linear mover 133b rises up by a relatively greater height than the third linear mover 133a, so that the end-effector 200 is lifted at the back side.

According to the descriptions of various operations above, the operation by the first actuator 110 may be directly performed with respect to the mounting plate 115 arranged above the first plate 111a, the operation by the second actuator 120 may be performed with respect to the first plate 111a of the first actuator 110, and the operation by the third actuator 130 may be performed with respect to the second plate 121a.

Here, the third actuator 130 may generate the vertical rising and falling operation and the pitching operation with respect to the first plate 111a, wherein the third actuator 130 may individually operate the first plate 111a, separately from the first actuator 110 and the second actuator 120.

Thus, separately from the operation of the first plate 111a according to the second actuator 120, the operation by the third actuator 130 may be simultaneously performed with respect to the first plate 111a, and thus, a combined operation of the second actuator 120 and the third actuator 130, that is, a combined operation of the rolling operation by the second actuator 120 and the vertical rising and falling operation and the pitching operation by the third actuator 130, may be performed in a combined way with respect to the first plate 111a.

The combined operation described above may be intactly performed with respect to the mounting plate 115 arranged above the first plate 111a. Here, while the mounting plate 115 may perform the combined movement according to the first plate 111a, the mounting plate 115 may separately perform the translational or shifting operation, on the first plate 111a, according to the first actuator 110.

Thus, the mounting plate 115 may cause the combined operation of four degrees of freedom including all operations described above including the shifting operation by the first actuator 110, the rolling operation by the second actuator 120, and the vertical rising and falling operation and the pitching operation by the third actuator 130.

As a result, the surgical tool 220 mounted on the mounting plate 115 generating the combined motions of four degrees of freedom, may become capable of controlling the combined motions of four degrees of freedom.

The surgical device capable of controlling the operations by the tool of four degrees of freedom may be used in a computer-based surgical system based on a surgical plan. Thus, it is possible to control all of the actuators described above, by using the surgical system, and thus, a surgeon may relatively more freely perform surgery according to a surgical plan, with less physical restrictions and surgical space limitations.

Therefore, according to the surgical device according to the present disclosure, surgery having a high degree of freedom may be possible, whereby a surgeon may have a relatively more free posture, and an end-effector may be operated to correspond to a position and a direction of a corresponding surgical site based on a pre-set surgical plan.

Although various embodiments of the present disclosure have been described in detail above, one of ordinary skill in the art may implement the present disclosure by modifying the present disclosure in various ways without deviating from the concept and the range of the present disclosure defined in the accompanying claims. Therefore, future changes in the embodiments of the present disclosure shall not deviate from the description of the present disclosure.

## Claims

1. A surgical device comprising: a mounting plate on which an end-effector comprising a surgical tool extending in a first direction is mounted, the mounting plate being placed on a plane parallel to both a first axis in the first direction and a second axis in a second direction, the second direction crossing the first direction;
a first actuator comprising a first plate, on which the mounting plate is mounted to be capable of a reciprocating movement in the second direction, the first actuator causing the mounting plate to perform a first motion in the second direction with respect to the first plate;
a second actuator configured to rotate the first plate with respect to the first axis in the first direction to generate rotation to cause a second motion of the end-effector mounted on the mounting plate; and
a third actuator configured to make the first plate vertically rise up and fall down in a direction of a third axis orthogonal to both the first axis and the second axis to cause a third motion of the end-effector according to the vertical rising up and falling down, and to rotate the first plate with respect to the second axis in the second direction to cause a fourth motion of the end-effector according to the rotation.

2. The surgical device of claim 1, wherein the first actuator comprises: a first motor configured to drive the mounting plate on the first plate; and a power transmission unit configured to transmit power of the first motor to operate the mounting plate through the first motor.

3. The surgical device of claim 2, wherein the power transmission unit comprises:
a first linear mover configured to convert rotary movement of the first motor into linear reciprocating movement; and a translational lever configured to transfer the linear movement of the first linear mover to the mounting plate on the first plate to cause translational movement of the mounting plate.

4. The surgical device of claim 3, wherein the linear movement from and of the first linear mover is performed in a direction crossing the plane of the mounting plate, and the translational lever is hinge-coupled to the mounting plate to convert the linear movement from and of the first linear mover into a direction of the translational movement of the mounting plate.

5. The surgical device of any one of claims 1 to 4, wherein the second actuator comprises:
a second plate rotatably hinge-coupled to the first plate;
a second motor mount integrally coupled to the second plate;
a second motor mounted on the second motor mount;
a second linear mover configured to convert rotational force of the second motor into linear reciprocating movement; and
a rolling lever of which both ends are connected to the second linear mover and the first plate, the rolling lever being configured to cause the second motion of the first plate on the second plate.

6. The surgical device of claim 5, wherein the first, the second, and the third actuators are arranged in a housing protecting the first to third actuators, and
the third actuator comprises:
a third plate, a position of which is fixed to the housing;
a third motor and a fourth motor, configured to cause the third motion and the fourth motion of the first plate;
a third motor mount configured to support the third motor and the fourth motor and integrally fixed to the third plate;
a third linear mover and a fourth linear mover connected to the third motor and the fourth motor, respectively, and configured to convert rotary movement into linear reciprocating movement; and
a third operating rod and a fourth operating rod connected to the third linear mover and the fourth linear mover, respectively, and configured to collaboratively cause the third motion and/or the fourth motion of the first plate.

7. The surgical device of claim 6, wherein the third operating rod is directly hinge-coupled to the first plate, and the fourth operating rod is, through a link lever, hinge-coupled to the first plate.

8. The surgical device of claims 1 to 3, wherein the first, the second, and the third actuators are arranged in a housing protecting the first to third actuators, and
the third actuator comprises:
a third plate, a position of which is fixed with respect to the housing;
a third motor and a fourth motor, configured to cause the third motion and the fourth motion of the first plate;
a third motor mount configured to support the third motor and the fourth motor and integrally fixed to the third plate;
a third linear mover and a fourth linear mover connected to the third motor and the fourth motor, respectively, and configured to convert rotary movement into linear reciprocating movement; and
a third operating rod and a fourth operating rod connected to the third linear mover and the fourth linear mover, respectively, and configured to collaboratively cause the third motion and/or the fourth motion of the first plate.

9. The surgical device of claim 8, wherein the third operating rod is directly hinge-coupled to the first plate, and the fourth operating rod is, through a link lever, hinge-coupled to the first plate.

10. The surgical device of claim 1, wherein the first motion is a right and left translational movement within a predetermined distance in the second direction,
the second motion is a rolling movement according to rotation within a predetermined angular range with respect to the first axis in the first direction,
the third motion is a vertical translational movement according to rising up and falling down within a predetermined vertical distance with respect to the third axis in the third direction, and
the fourth motion is a pitching movement according to rotation within a predetermined angle with respect to the second axis in the second direction,
wherein the third motion and the fourth motion are performed individually or in combination, regardless of the first motion and the second motion, so that the end-effector is configured to perform any one motion from among the first motion to the fourth motion or a combined motion combining at least two motions from among the first motion to the fourth motion.

11. A method of controlling a surgical device, the method comprising: mounting an end-effector comprising a tool extending in a first direction on a mounting plate placed on a plane parallel to both a first axis in the first direction and a second axis in a second direction, the second direction crossing the first direction;
causing, by a first actuator comprising a first plate, the mounting plate on the first plate to perform a first motion in the second direction;
causing, by a second actuator, by rotating the first actuator with respect to the first axis in the first direction, a second motion of the end-effector on the mounting plate mounted on the first plate;
causing, by a third actuator, a third motion of the end effector, in combination with the first motion and the second motion of the end effector that are controlled by the first actuator and the second actuator, by vertically moving the first plate of the first actuator along a third axis in a third direction orthogonal to the first direction and the second direction; and
causing, by the third actuator, a fourth motion of the end effector, in combination with the first motion and the second motion of the end effector that are controlled by the first actuator and the second actuator, by rotating the first plate of the first actuator with respect to the second axis in the second direction.

12. The method of claim 11, wherein a first motor provided in the first actuator is configured to generate rotational power,
a first linear mover provided in the first actuator performs a linear reciprocating movement through the rotational power, and
a translational lever provided in the first actuator is operated by the linear reciprocating movement to cause a reciprocating movement of the mounting plate in the second direction, thereby causing the first motion of the end-effector.

13. The method of claim 11 or 12, wherein a second motor of the second actuator is configured to generate rotational power, and
a second linear mover of the second actuator performs a linear reciprocating movement through the rotational power to control an inclination of the first plate on a second plate of the second actuator, thereby causing the second motion of the end-effector.

14. The method of claim 13, wherein each of a third motor and a fourth motor, provided in the third actuator, is configured to generate rotational power, and
each of a third linear mover and a fourth linear mover, provided in the third actuator, performs a linear reciprocating movement through the rotational power to make the first plate on a third plate provided in the third actuator rise up and fall down in the third direction, thereby causing any one of the third motion and the fourth motion of the end-effector.

15. The method of claim 11 or 12, wherein each of a third motor and a fourth motor, provided in the third actuator, is configured to generate rotational power, and
each of a third linear mover and a fourth linear mover, provided in the third actuator, performs a linear reciprocating movement through the rotational power to make the first plate on a third plate provided in the third actuator rise up and fall down in the third direction, thereby causing any one of the third motion and the fourth motion of the end-effector.

16. The method of claim 15, wherein the third motion is performed according to operations of the third linear mover and the fourth linear mover of a same distance, and
the fourth motion is performed according to operations of the third linear mover and the fourth linear mover of different distances.

17. The method of claim 14, wherein the third motion is performed according to operations of the third linear mover and the fourth linear mover of a same distance, and
the fourth motion is performed according to operations of the third linear mover and the fourth linear mover of different distances.

## Amended claims

### Amended claims under Art. 19.1 PCT

1. A surgical device comprising: a mounting plate on which an end-effector comprising a surgical tool extending in a first direction is mounted, the mounting plate being placed on a plane parallel to both a first axis in the first direction and a second axis in a second direction, the second direction crossing the first direction;
a first actuator comprising a first plate, on which the mounting plate is mounted to be capable of a reciprocating movement in the second direction, the first actuator causing the mounting plate to perform a first motion in the second direction with respect to the first plate;
a second actuator configured to rotate the first plate with respect to the first axis in the first direction to generate rotation to cause a second motion of the end-effector mounted on the mounting plate; and
a third actuator configured to make the first plate vertically rise up and fall down in a direction of a third axis orthogonal to both the first axis and the second axis to cause a third motion of the end-effector according to the vertical rising up and falling down, and to rotate the first plate with respect to the second axis in the second direction to cause a fourth motion of the end-effector according to the rotation.

2. The surgical device of claim 1, wherein the first actuator comprises: a first motor configured to drive the mounting plate on the first plate; and a power transmission unit configured to transmit power of the first motor to operate the mounting plate through the first motor.

3. The surgical device of claim 2, wherein the power transmission unit comprises:
a first linear mover configured to convert rotary movement of the first motor into linear reciprocating movement; and a translational lever configured to transfer the linear movement of the first linear mover to the mounting plate on the first plate to cause translational movement of the mounting plate.

4. The surgical device of claim 3, wherein the linear movement from and of the first linear mover is performed in a direction crossing the plane of the mounting plate, and the translational lever is hinge-coupled to the mounting plate to convert the linear movement from and of the first linear mover into a direction of the translational movement of the mounting plate.

5. The surgical device of claim 1, wherein the second actuator comprises:
a second plate rotatably hinge-coupled to the first plate;
a second motor mount integrally coupled to the second plate;
a second motor mounted on the second motor mount;
a second linear mover configured to convert rotational force of the second motor into linear reciprocating movement; and
a rolling lever of which both ends are connected to the second linear mover and the first plate, the rolling lever being configured to cause the second motion of the first plate on the second plate.

6. The surgical device of claim 5, wherein the first, the second, and the third actuators are arranged in a housing protecting the first to third actuators, and
the third actuator comprises:
a third plate, a position of which is fixed to the housing;
a third motor and a fourth motor, configured to cause the third motion and the fourth motion of the first plate;
a third motor mount configured to support the third motor and the fourth motor and integrally fixed to the third plate;
a third linear mover and a fourth linear mover connected to the third motor and the fourth motor, respectively, and configured to convert rotary movement into linear reciprocating movement; and
a third operating rod and a fourth operating rod connected to the third linear mover and the fourth linear mover, respectively, and configured to collaboratively cause the third motion and/or the fourth motion of the first plate.

7. The surgical device of claim 6, wherein the third operating rod is directly hinge-coupled to the first plate, and the fourth operating rod is, through a link lever, hinge-coupled to the first plate.

8. The surgical device of claim 1, wherein the first, the second, and the third actuators are arranged in a housing protecting the first to third actuators, and
the third actuator comprises:
a third plate, a position of which is fixed with respect to the housing;
a third motor and a fourth motor, configured to cause the third motion and the fourth motion of the first plate;
a third motor mount configured to support the third motor and the fourth motor and integrally fixed to the third plate;
a third linear mover and a fourth linear mover connected to the third motor and the fourth motor, respectively, and configured to convert rotary movement into linear reciprocating movement; and
a third operating rod and a fourth operating rod connected to the third linear mover and the fourth linear mover, respectively, and configured to collaboratively cause the third motion and/or the fourth motion of the first plate.

9. The surgical device of claim 8, wherein the third operating rod is directly hinge-coupled to the first plate, and the fourth operating rod is, through a link lever, hinge-coupled to the first plate.

10. The surgical device of claim 1, wherein the first motion is a right and left translational movement within a predetermined distance in the second direction,
the second motion is a rolling movement according to rotation within a predetermined angular range with respect to the first axis in the first direction,
the third motion is a vertical translational movement according to rising up and falling down within a predetermined vertical distance with respect to the third axis in the third direction, and
the fourth motion is a pitching movement according to rotation within a predetermined angle with respect to the second axis in the second direction,
wherein the third motion and the fourth motion are performed individually or in combination, regardless of the first motion and the second motion, so that the end-effector is configured to perform any one motion from among the first motion to the fourth motion or a combined motion combining at least two motions from among the first motion to the fourth motion.

11. A method of controlling a surgical device, the method comprising: mounting an end-effector comprising a tool extending in a first direction on a mounting plate placed on a plane parallel to both a first axis in the first direction and a second axis in a second direction, the second direction crossing the first direction;
causing, by a first actuator comprising a first plate, the mounting plate on the first plate to perform a first motion in the second direction;
causing, by a second actuator, by rotating the first actuator with respect to the first axis in the first direction, a second motion of the end-effector on the mounting plate mounted on the first plate;
causing, by a third actuator, a third motion of the end effector, in combination with the first motion and the second motion of the end effector that are controlled by the first actuator and the second actuator, by vertically moving the first plate of the first actuator along a third axis in a third direction orthogonal to the first direction and the second direction; and
causing, by the third actuator, a fourth motion of the end effector, in combination with the first motion and the second motion of the end effector that are controlled by the first actuator and the second actuator, by rotating the first plate of the first actuator with respect to the second axis in the second direction.

12. The method of claim 11, wherein a first motor provided in the first actuator is configured to generate rotational power,
a first linear mover provided in the first actuator performs a linear reciprocating movement through the rotational power, and
a translational lever provided in the first actuator is operated by the linear reciprocating movement to cause a reciprocating movement of the mounting plate in the second direction, thereby causing the first motion of the end-effector.

13. The method of claim 11, wherein a second motor of the second actuator is configured to generate rotational power, and
a second linear mover of the second actuator performs a linear reciprocating movement through the rotational power to control an inclination of the first plate on a second plate of the second actuator, thereby causing the second motion of the end-effector.

14. The method of claim 13, wherein each of a third motor and a fourth motor, provided in the third actuator, is configured to generate rotational power, and
each of a third linear mover and a fourth linear mover, provided in the third actuator, performs a linear reciprocating movement through the rotational power to make the first plate on a third plate provided in the third actuator rise up and fall down in the third direction, thereby causing any one of the third motion and the fourth motion of the end-effector.

15. The method of claim 11, wherein each of a third motor and a fourth motor, provided in the third actuator, is configured to generate rotational power, and
each of a third linear mover and a fourth linear mover, provided in the third actuator, performs a linear reciprocating movement through the rotational power to make the first plate on a third plate provided in the third actuator rise up and fall down in the third direction, thereby causing any one of the third motion and the fourth motion of the end-effector.

16. The method of claim 15, wherein the third motion is performed according to operations of the third linear mover and the fourth linear mover of a same distance, and
the fourth motion is performed according to operations of the third linear mover and the fourth linear mover of different distances.

17. The method of claim 14, wherein the third motion is performed according to operations of the third linear mover and the fourth linear mover of a same distance, and
the fourth motion is performed according to operations of the third linear mover and the fourth linear mover of different distances.

Statement under Art. 19.1 PCT
In accordance with Article 19 of the Patent Cooperation Treaty (PCT), the claims are amended as follows in response to the Written Opinion issued by the International Searching Authority.

**1. Amendments in Response to PCT Rule 43.2.1(a)(i): Typographical Errors**

The following typographical errors in the claims have been corrected for clarity and consistency:
 Claim 4: " 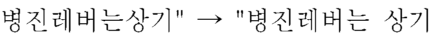 "
 Claim 5: " 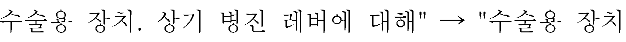 "
 Claim 6: " 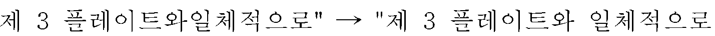 "
 Claims 7 and 9: " 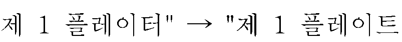 "
 Claim 12: " 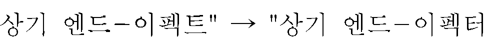 "

These corrections do not change the substance of the claimed invention and are solely intended to eliminate clerical inconsistencies.

**2. Amendment in Response to PCT Rule 6.4(a): Dependency Structure**

Claim 8 was found to violate PCT Rule 6.4(a) by failing to indicate alternative dependencies properly. To comply with the formal requirements of the PCT, Claim 8 has been amended into a singly dependent claim.

Additionally, Claims 5, 11, and 15 have been voluntarily amended into singly dependent form, not due to any violation under the PCT, but in consideration of the requirements and practices of certain designated Offices in the national phase. These amendments are intended to ensure procedural clarity and facilitate smooth prosecution.

These amendments are made solely in response to the formal objections raised in the Written Opinion and do not introduce any new subject matter beyond the contents of the originally filed international application.
